# EUROPEAN PATENT APPLICATION

(11) **EP 4 806 436 A1**
(43) Date of publication of application: **16.09.2026**
(21) Application number: 24887831.6
(22) Date of filing: 30.10.2024
(51) Int. Cl.: A61K 31/4965, A61P 21/00

(54) **USE OF LIGUSTRAZINE NITRONE DERIVATIVE IN PREPARATION OF DRUG FOR PREVENTING OR TREATING SARCOPENIA**

(30) Priority: 08.11.2023 CN 202311473401
(71) Applicant: Guangzhou Magpie Pharmaceuticals Co., Ltd., Guangzhou, Guangdong 510005 (CN)
(72) Inventor: JING, Mei, Guangzhou, Guangdong 510005 (CN)
(74) Representative: Eisenführ Speiser
(86) International application number: PCT/CN2024/128563
(87) International publication number: WO 2025/098219

(57) **Abstract**

The present invention provides the use of a ligustrazine nitrone derivative or a pharmaceutically acceptable salt thereof in the manufacture of medicaments for the prevention or treatment of sarcopenia. The ligustrazine nitrone derivative can effectively improve muscle mass, muscle strength, and muscle function in sarcopenia model animals, and has the potential to be developed into a preventive or therapeutic drug for sarcopenia. The ligustrazine nitrone derivative has a structure of the following formula (I):

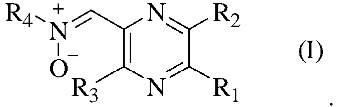

## Description

### FIELD OF THE INVENTION

The present invention relates generally to the field of medicine and, more particularly, to a novel use of a ligustrazine nitrone derivative or a pharmaceutically acceptable salt thereof in the manufacture of medicaments for the prevention or treatment of sarcopenia.

### BACKGROUND OF THE INVENTION

Sarcopenia is a syndrome characterized by age-related muscle mass loss, decreased strength, and/or decreased physical function. As muscles weaken, accompanied by a decrease in mobility, it can lead to slow movement, poor balance, and an increased risk of falling. Epidemiological investigation found that with increase of age, the incidence rate of sarcopenia increased year by year, and increased sharply after the age of 50. The incidence rate of sarcopenia is 15% in elderly people aged 65 and above, and as high as 50% in the population aged 80 and above (Cohen S, Nathan J A, Goldberg A L. Muscle wasting in disease: molecular mechanisms and promising therapies[J]. Nat Rev Drug Discov, 2015, 14(1): 58-74). Sarcopenia is characterized by high incidence rate, complicated etiology, concealed progress, and extensive adverse effects. However, current research both domestically and internationally has not fully revealed its pathogenesis, let alone specific medicines. The clinical practice mainly involves nutritional intervention combined with impedance exercise. However, for most elderly people, such as those in nursing homes and hospitals, impedance exercise is difficult to implement. It is urgent to develop therapeutic drugs for sarcopenia to meet the needs of clinical treatments.

### SUMMARY OF THE INVENTION

In order to solve the problems in the existing technology, the present invention provides a use of a ligustrazine nitrone derivative or a pharmaceutically acceptable salt thereof in the prevention or treatment of sarcopenia and the manufacture of the corresponding medicament.

The present invention provides a use of the ligustrazine nitrone derivative or a pharmaceutically acceptable salt thereof in the prevention or treatment of sarcopenia, wherein, the use comprises administering to a patient in need a therapeutically effective amount of the ligustrazine nitrone derivative or a pharmaceutically acceptable salt thereof.

The present invention provides also the use of the ligustrazine nitrone derivative or a pharmaceutically acceptable salt thereof in the manufacture of a medicament for the prevention or treatment of sarcopenia.

The ligustrazine nitrone derivative of the present invention has a structure of the following formula (I): 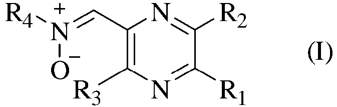 wherein,
R₁ is hydrogen, methyl or 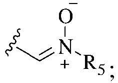
R₂ and R₃ are each independently hydrogen or a C₁-C₆ alkyl; R₄ is sec-butyl, isobutyl, tert-butyl, cyclopentyl, or cyclohexyl; R₅ is sec-butyl, isobutyl, tert-butyl, cyclopentyl, or cyclohexyl.

Preferably, R₂ and R₃ are each independently hydrogen, methyl, ethyl, n-propyl, isopropyl, n-butyl, isobutyl, tert-butyl, or n-pentyl; more preferably, R₂ and R₃ are each independently methyl, ethyl, or propyl.

According to an embodiment of the present invention, the ligustrazine nitrone derivative has a structure of the following formula, TBN or TN-2:

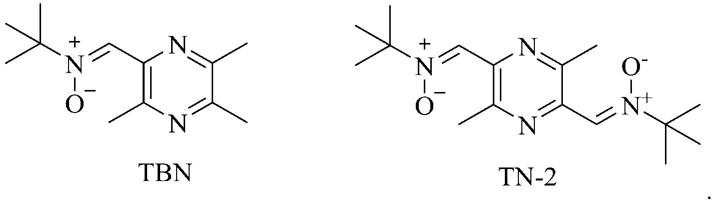

According to an embodiment of the present invention, sarcopenia is primary sarcopenia or secondary sarcopenia. Primary sarcopenia is mainly age-related. Secondary sarcopenia is mainly related to diseases (such as metabolic disorders, chronic heart, lung, liver, kidney diseases, and cerebrovascular diseases), drug abuse, reduced physical activity, unhealthy lifestyle, etc.

According to an embodiment, the present invention provides the use of a ligustrazine nitrone derivative or a pharmaceutically acceptable salt thereof in the preparation of a medicament for slowing down the reduction in muscle mass caused by sarcopenia, maintaining or increasing muscle mass.

According to an embodiment, the present invention provides the use of a ligustrazine nitrone derivative or a pharmaceutically acceptable salt thereof in the preparation of a medicament for improving muscle strength in patients with sarcopenia.

According to an embodiment, the present invention provides the use of a ligustrazine nitrone derivative or a pharmaceutically acceptable salt thereof in the preparation of a medicament for improving physical function in patients with sarcopenia.

According to an embodiment, the present invention provides the use of a ligustrazine nitrone derivative or a pharmaceutically acceptable salt thereof in the preparation of a medicament for treating or preventing muscle fibrosis in sarcopenia.

According to an embodiment, the pharmaceutically acceptable salt of the present invention may be a salt formed with an inorganic acid, wherein the inorganic acid is for example hydrochloric acid, hydrobromic acid, hydroiodic acid, sulfuric acid, pyrosulfuric acid, phosphoric acid, or nitric acid; or a salt formed with an organic acid, wherein the organic acid is for example methanesulfonic acid, ethanesulfonic acid, benzenesulfonic acid, p-toluenesulfonic acid, acetic acid, pyruvic acid, trifluoroacetic acid, propionic acid, hexanoic acid, benzoic acid, salicylic acid, cinnamic acid, cyclopentanone propionic acid, dodecyl sulfuric acid, 2-naphthalenesulfonic acid, citric acid, tartaric acid, stearic acid, lactic acid, oxalic acid, succinic acid, malic acid, fumaric acid, D-gluconic acid, or aspartic acid.

The ligustrazine nitrone derivative or a pharmaceutically acceptable salt thereof of the present invention can be administered orally or parenterally, such as rectal, local, transdermal, intravenous, intramuscular, intraperitoneal, or subcutaneous administrations. The dosage forms used for oral administration may include, but are not limited to, tablets, pills, soft or hard capsules, granules, powders, micro powders, liquids, lotion or micro pills. The dosage forms used for parenteral administration may include, but are not limited to, eye drops, injections, medicinal drops, lotions, ointments, gel, creams, suspensions, emulsions, suppositories, patches or spray.

The dosage of the ligustrazine nitrone derivative or a pharmaceutically acceptable salt thereof according to the present invention can be calculated as 10-5000 mg/person/time based on the ligustrazine nitrone derivative. The specific dosage may vary depending on the age, gender, weight, specific pathological condition and its severity, administration route or diagnosis of the subject. The dosage of medication based on the above factors can be determined according to the level of technical personnel in this field. The specific method and frequency of administration can follow the common method of administration of the ligustrazine nitrone derivative (such as TBN or TN-2), for example, they can be administered once or multiple times a day.

The present invention provides the use of a ligustrazine nitrone derivative or a pharmaceutically acceptable salt thereof in the preparation of drugs for the prevention or treatment of sarcopenia. In a study on the effect of the ligustrazine nitrone derivative TBN on D-galactose-induced sarcopenia mouse model, the present invention showed that TBN can improve the wet to weight ratio of gastrocnemius muscle and effectively reduce the degree of gastrocnemius muscle fibrosis in the sarcopenia mouse model. In addition, the ligustrazine nitrone derivative TBN can significantly prolong the pole climbing time induced in mice by D-galactose, increase their grip and reduce the time it takes for the spinning rod to fall, and significantly improve the muscle strength of the mice with sarcopenia. At the same time, TBN can increase the moving speed, swing speed, step cadence, and stride length of D-galactose-induced sarcopenia mice, reduce the passage time of mice through equidistant monitoring corridors, and effectively improve the physical coordination ability of sarcopenia mice. The ligustrazine nitrone derivative of the present invention can effectively improve muscle mass, muscle strength, and physical function in sarcopenia model animals, and has the potential to be developed into a preventive or therapeutic drug for sarcopenia.

### BRIEF DESCRIPTION OF THE DRAWINGS

Figure 1 shows the effect of different doses of TBN on the body weight of the D-galactose-induced sarcopenia mice, with the horizontal axis representing time (week) and the vertical axis representing body weight (g).
Figure 2 shows the effect of different doses of TBN on the food intake of the D-galactose-induced sarcopenia mice, with the horizontal axis representing time (week) and the vertical axis representing food intake (g).
Figure 3 shows the results of different doses of TBN on the ratio of the wet weight of gastrocnemius muscle to the body weight in the D-galactose-induced sarcopenia mice, with horizontal axis representing grouping, and the vertical axis representing the ratio of gastrocnemius wet weight/body weight (mg/g).
Figure 4 shows the effect of TBN on pole climbing time in the D-galactose-induced sarcopenia mice during pole climbing experiments, with horizontal axis representing grouping, and the vertical axis representing the time (s) from pole top to bottom platform.
Figure 5 shows the effect of TBN on the grasping force of limbs in the D-galactose-induced sarcopenia mice, with the horizontal axis representing grouping and the vertical axis representing grasping force values (Strength, g).
Figure 6 shows the effect of TBN on the drop time of the D-galactose-induced sarcopenia mice during the rod rotation experiment, with the horizontal axis representing grouping and the vertical axis representing time (s).
Figure 7 shows the representative gait image of mice in each group during the gait experiment of the D-galactose-induced sarcopenia mice treated with TBN.
Figure 8 shows the effect of TBN on the stride length in the D-galactose-induced sarcopenia mice, with the horizontal axis representing grouping of different limbs and the vertical axis representing stride length (cm).
Figure 9 shows the effect of TBN on the step cadence in the D-galactose-induced sarcopenia mice, with the x-axis representing grouping and the y-axis representing cadence.
Figure 10 shows the effect of TBN on the percentage of diagonal support time in the D-galactose-induced sarcopenia mice, with the horizontal axis representing grouping and the vertical axis representing percentage of the time of diagonal support (%).
Figure 11 shows the effect of TBN on the run duration of the D-galactose-induced sarcopenia mice, with the horizontal axis representing grouping and the vertical axis representing run duration (s).
Figure 12 shows the effect of TBN on the stand time of the D-galactose-induced sarcopenia mice, with the horizontal axis representing grouping and the vertical axis representing stand time (s).
Figure 13 shows the effect of TBN on the run average speed of the D-galactose-induced sarcopenia mice, with the horizontal axis representing grouping and the vertical axis representing run average speed (cm/s).
Figure 14 shows the effect of TBN on the swing speed in the D-galactose-induced sarcopenia mice, with the horizontal axis representing grouping and the vertical axis representing swing speed (cm/s).
Figure 15 is a representative image of TBN on the positive staining for HE, Masson, and α-SMA in the D-galactose-induced sarcopenia mice.
Figure 16 shows the statistical results of area of muscle fibrosis in Masson staining, with the horizontal axis representing grouping and the vertical axis representing the percentage of area of fibrosis (%).
Figure 17 is a statistical chart of α-SMA positive area, with the horizontal axis representing grouping and the vertical axis representing the percentage of the area of α-SMA positive (%).

In the accompanying drawings of the present invention: ***P<0.001, **P<0.01, *P<0.05 vs. model group.

### DETAILED DESCRIPTION OF THE EMBODIMENTS

The following embodiments facilitate a better understanding of the present invention, but do not limit the scope of the present invention. The experimental methods in the following examples, unless otherwise specified, are all conventional methods. The test materials used in the following examples, unless otherwise specified, were purchased from conventional biochemical reagent stores.

### Example 1

### 1. Preparation of sarcopenia model

The muscle atrophy model in this embodiment is induced using D-galactose. The experimental animals are induced by intraperitoneal injection of 200 mg/kg D-galactose at 6 pm every day (for 8 weeks), while the control group animals are induced by intraperitoneal injection of an equal amount of physiological saline at 6 pm every day.

### 2. Grouping and administration methods

The combination of dasatinib and quercetin is administered as a positive control drug, with dasatinib of 5 mg/kg per dose, quercetin of 50 mg/kg per dose, and TBN of 10 mg/kg, 30 mg/kg and 60 mg/kg per dose. The specific grouping and dosage are shown in Table 1.

**Table 1. Dose design for this experiment**

| No. | Group | Dose (mg/kg) | Administration volume (ml/kg) | Number of mice (male) |
|---|---|---|---|---|
| 1 | Control group (Ctrl) | 0 | 10 | 12 |
| 2 | Model group (Model) | 0 | 10 | 12 |
| 3 | TBN low-dose group | 10 | 10 | 12 |
| 4 | TBN medium-dose group | 30 | 10 | 12 |
| 5 | TBN high-dose group | 60 | 10 | 12 |
| 6 | Dasatinib+Quercetin group (D+Q group) | 5+50 | 10 | 12 |

Intragastric administration. After the start of D-galactose-induced modeling, animals in each group are administered by gavage starting from the 15th day. The control group, model group, TBN low-dose group, TBN medium-dose group, and TBN high-dose group are administered by gavage once a day at 9 am. The dasatinib+quercetin group is administered continuously for 3 days every two weeks, and each group of theanimals is administered continuously for 6 weeks.

### 3. Effect evaluation

The improvement effect of TBN treatment on motor function in sarcopenia mice is evaluated through a series of behavioral tests such as pole climbing, spinning, grip, and gait experiments. The effect of TBN on muscle fibrosis in sarcopenia mice is evaluated through HE staining, Masson staining, and α-SMA.

The data is analyzed using Graphpad Prism9.0 software, and the data is represented by Mean ± SEM. Comparisons between multiple groups are conducted using one-way ANOVA or two-way ANOVA test methods, with p<0.05 considered to have significant statistical differences.

### 3.1 Influence of the ratio of body weight and gastrocnemius muscle wet weight

Starting from before the experimental modeling, the weekly weight and food intake changes of mice are recorded, and when sampling, the weight and wet weight of the gastrocnemius muscle of the mice are recorded until the end of behavioral studies.

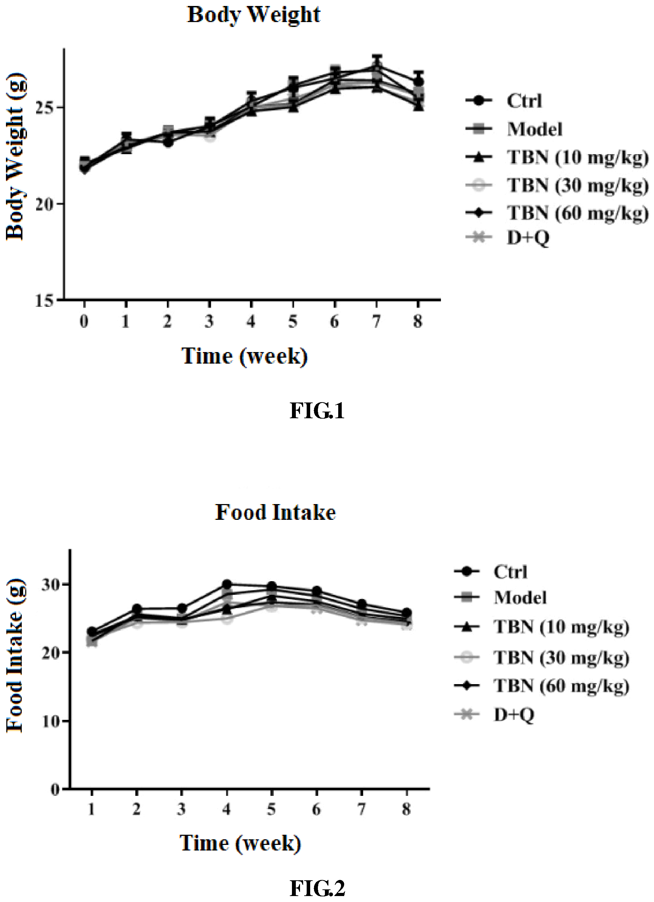

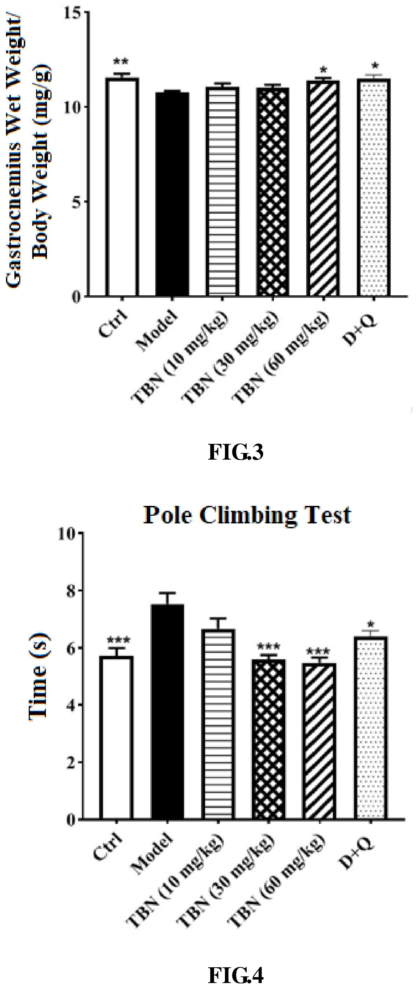

The experimental results showed that there is no significant difference in body weight (Figure 1) and food intake (Figure 2) among the groups of mice, indicating that TBN treatment had no significant effect on the body weight and food intake of mice; the ratio of wet weight gastrocnemius muscle to body weight in the D-galactose-induced sarcopenia mice is significantly reduced compared to the control group, while high-dose TBN treatment increased the ratio of wet weight of gastrocnemius muscle to body weight in mice (Figure 3).

### 3.2 Assessment of motor function

### 3.2.1 Pole climbing test

After 6 weeks of administration, the limb movement ability and muscle strength of the mice are tested via a pole climbing test. This experiment involves climbing a wooden pole with a length of 60 cm and a diameter of approximately 1 cm. The pole is wrapped with medical tape to increase the friction between the mouse and the pole. During the experiment, the tail of the mouse is grasped with its head facing downwards, the tail is released, and time starts. The time is recorded for the mice to crawl to the bottom of the pole without external force, based on the landing of the forelimb. The maximum testing time is 15 seconds, and, if exceeded 15 seconds, it will be counted as 15 seconds. After 6 weeks of administration, pole climbing training is conducted. After three consecutive training sessions, mice that did not meet the standards are excluded. On the second day of training, pole climbing experiments are conducted, with each mouse tested three times to obtain the average value.

### 3.2.2 Rod rotation test

After 6 weeks of administration, the rod rotation test is conducted to evaluate the motor coordination of mice in a rotating state. A protruding plastic round wood rod (diameter 6.0 cm) is fixed at a height of 30 cm. The time of falling is automatically recorded by the instrument. Before testing, mice are trained at a speed of 10 rpm/s for 3 days, lasting for 3 minutes. After the training, each mouse is subjected to 3 experiments on accelerated rotation (40 rpm per minute). When the mouse cannot stay on the stick for 3 minutes, then the average falling time is recorded. The average dropping time of three mouse is calculated as the duration of the mouse's stay on the rotating rod.

### 3.2.3 Grasping test

After 6 weeks of administration, the grasping test is conducted to directly evaluate the strength of mouse limb muscles. The mouse is placed in the center of the grasping plate of a grasper and its tail is gently pulled to encourage it to grasp the grasping plate. When the mouse firmly grasps the grasping plate, it is immediately pulled back until releasing its claws, which indicates the maximum grasping strength of the mouse. The experimental process is repeated three times, and the average of the three results is taken as the evaluation value.

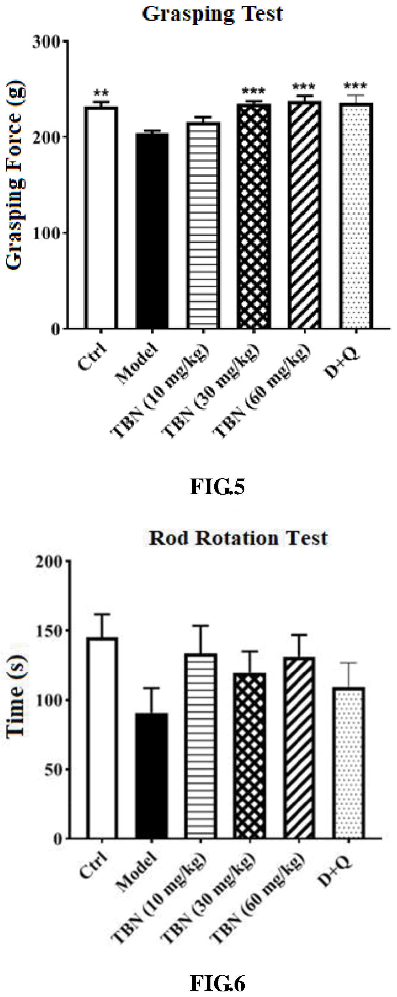

As shown in Figures 4 to 6, the D-galactose-induced sarcopenia mice showed an increase in pole climbing time (Figure 4), a decrease in grip strength (Figure 5), and a shorter time to spin and drop (Figure 6) compared to the control group mice, indicating a decrease in motor function induced by D-galactose. However, the low-dose, medium-dose, and high-dose groups of TBN are able to significantly improve the D-galactose-induced motor function.

### 3.2.4 Gait test

After 6 weeks of administration, gait analysis is used to evaluate gait performance and identify abnormal movements in mice. Gait data collection and analysis are conducted using a CatWalk XT system (Noldus). The system consists of a 1.3-meter-long black corridor and a 70 centimeter thick glass panel. Under the mouse walkway, a high-speed camera is used to capture the outline of mouse paw prints. According to the manufacturer's recommendation, the area of the aisle is set to 10×20 centimeters. Three days before the test, each mouse walked freely on a glass plate at least five times a day to adapt to this gait environment before the test. During testing, we set the minimum and maximum running times to 1 second and 10 seconds respectively, and the data of each mouse is collected three times. For all groups of mice, the same detection settings are used (camera gain: 20.0, green intensity threshold: 0.10, red ceiling light: 17.7, green channel light: 16.5). For analysis, after the gait parameters are automatically generated, each footprint is manually checked and labeled with LF (left front), LH (left rear), RF (right front), and RH (right rear) claws. Gait parameters such as movement speed, swing speed, standing and swing time, stride length, step cadence, and diagonal support time are collected.

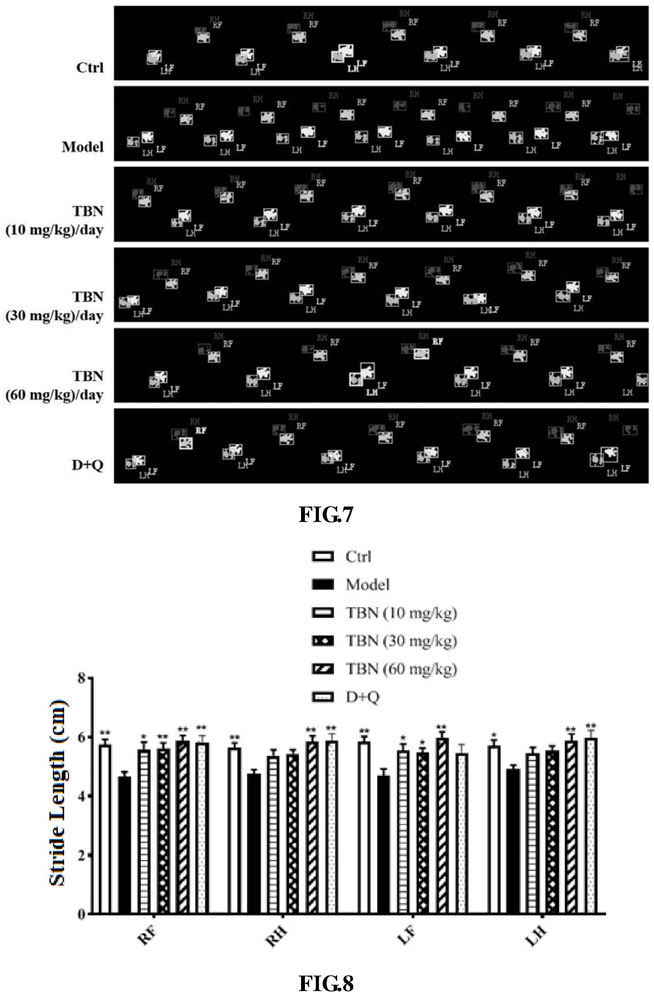

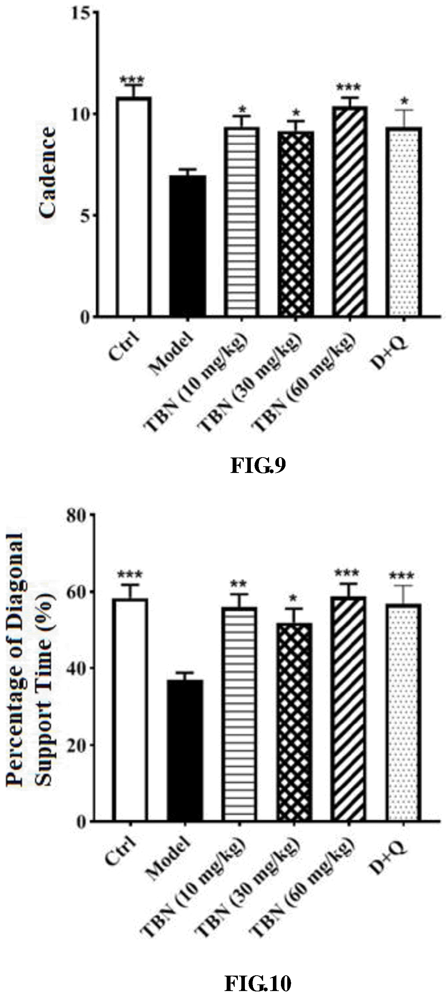

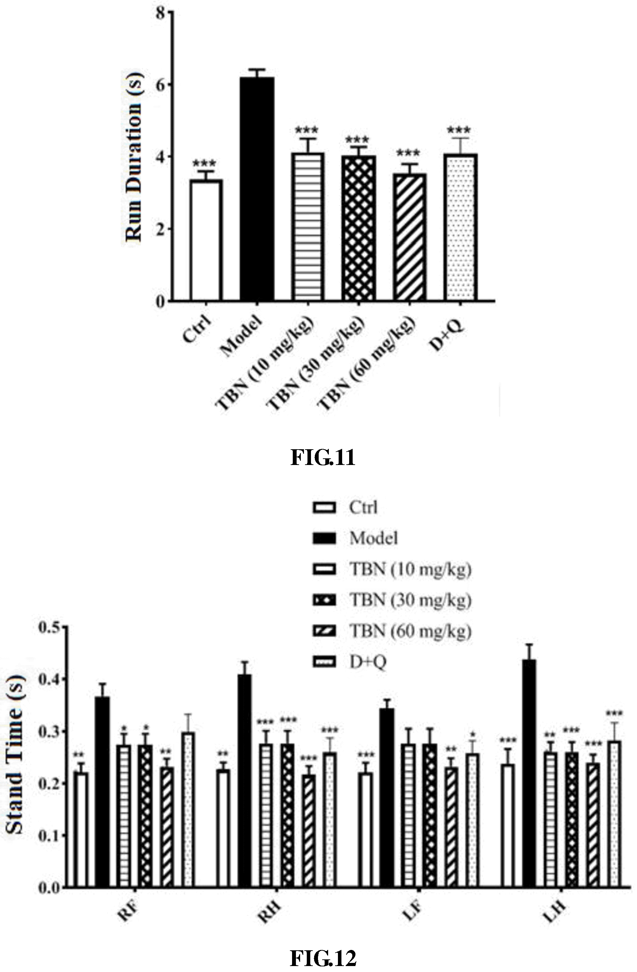

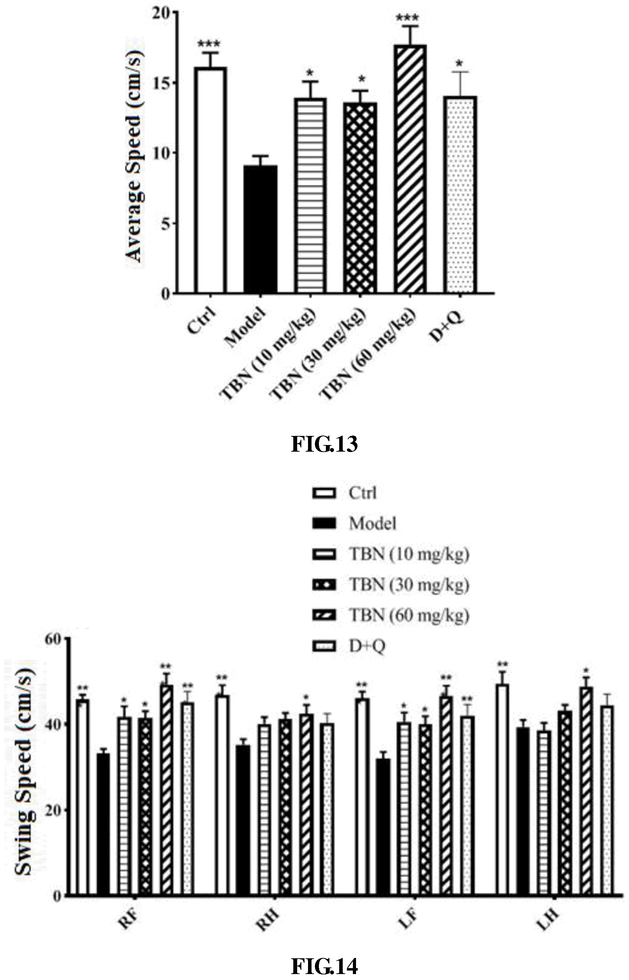

In the gait test on the effect of TBN to the D-galactose-induced sarcopenia mice, the gait of each group of mice is shown in Figure 7, the stride length (cm) is shown in Figure 8, the cadence is shown in Figure 9, the time of diagonal support (%) is shown in Figure 10, the run duration (s) of gait is shown in Figure 11, the stand time (s) is shown in Figure 12, the run average speed (cm/s) is shown in Figure 13, and the swing speed (cm/s) is shown in Figure 14. The results showed that TBN treatment could increase the run average speed, swing speed, stride length, and cadence of the D-galactose-induced sarcopenia mice, reduce the run duration and stand time of mice passing through equidistant monitoring corridors, indicating that TBN can improve the motor coordination ability of sarcopenia mice.

### 3.3 Assessment of muscle fibrosis

### 3.3.1 HE staining

Deeply anesthetize mice with 4% chloral hydrate are perfused with physiological saline, and dissected, the samples are collected. The right gastrocnemius muscle is dehydrated overnight at 4°C in fresh 4% paraformaldehyde using an alcohol gradient (75% ethanol, 2 hours; 85% ethanol, 1 hour; 95% ethanol, 1 hour; 95% ethanol, 1 hour; 100% ethanol, 0.5 hours; 100% ethanol) and prepared for embedding in paraffin. 5 µm paraffin slices of gastrocnemius muscle tissue are prepared for histological analysis.

The paraffin slices are first deparaffinized, soaked in xylene, and then rehydrated in gradient alcohol. The slices are stained with hematoxylin for 10 minutes, rinsed with tap water for 10 minutes, and stained with eosin for 1 minute. The slices are rinsed thoroughly in tap water, made transparent with ethanol and xylene, and finally covered with a glass slide. The stained slices are examined under a microscope and the images are captured using a Leica Aperio GT 450 scanner (Leica, Germany).

### 3.3.2 Masson staining

The paraffin slices are first deparaffinized, soaked in xylene, and then rehydrated in gradient alcohol. The dewaxed and rehydrated slices are immersed in Bouin solution at room temperature overnight and rinsed with water until the yellow color faded away; drip dyed with celadon blue staining solution for 2 minutes, then washed with water; drip dyed with Mayer hematoxylin staining solution for 2 minutes, and then rinsed with clean water; drip dyed with acidic ethanol solution for 10 seconds, rinsed with running water for 10 minutes; stained with Lichun Hongfuchsin staining solution for 10 minutes, rinsed with clean water; treated with phosphomolybdic acid solution for about 10 minutes; drip dyed with aniline blue solution for 5 minutes; weak acid differentiated for 2 minutes; dehydrated with gradient ethanol, after transparency with xylene the film is sealed, and the images are captured using a Leica Aperio GT 450 scanner (Leica, Germany).

### 3.3.3 α-SMA immunohistochemical staining

Paraffin slices are first deparaffinized, soaked in xylene, and then rehydrated in gradient alcohol. The muscle tissue slices that have been dewaxed and rehydrated are washed (three times with PBS), and repaired with antigen repair solution (1×), soaked in PBS three times; incubated in hydrogen peroxide for 10 minutes to remove endogenous peroxidase, and washed three times with PBS; incubated with 3% BSA (containing 0.3% Triyon-100) at room temperature for 1 hour; incubated with α-SMA (1:500) antibody at 4 °C overnight and washed with PBS 4 times; incubated with secondary antibody working solution at room temperature for 1 hour and washed with PBS 4 times; incubated with a three antibody working solution for 1 hour and washed with PBS 4 times; stained with DAB for 2 minutes, immersed with PBS; and, after dehydration and transparency of xylene, the film is sealed and images are captured using a Leica Aperio GT 450 scanner (Leica, Germany).

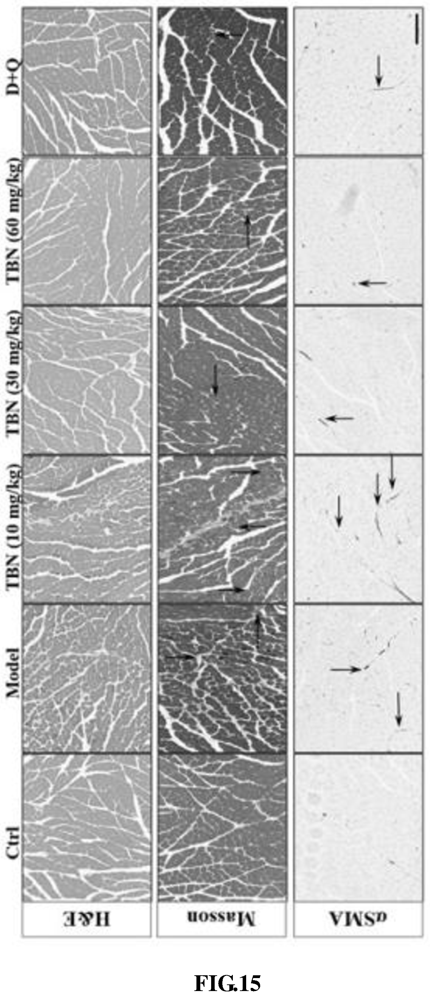

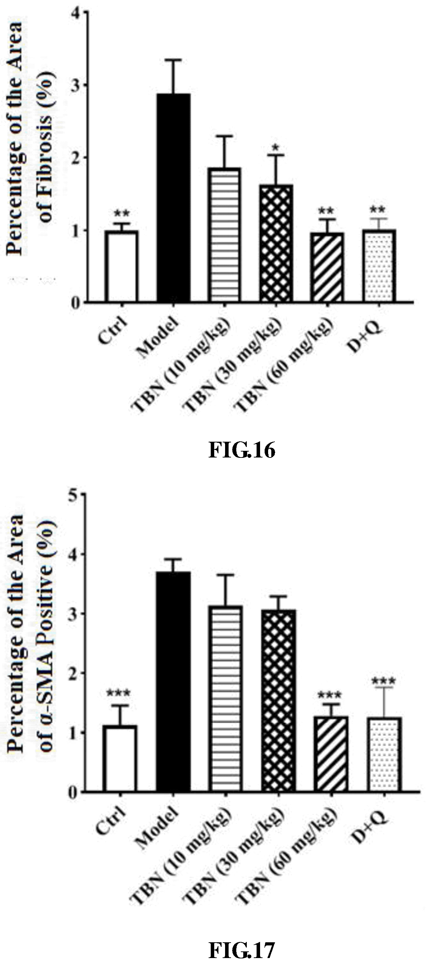

Results: Representative images of HE staining, Masson staining, and α-SMA immunohistochemistry staining in the gastrocnemius muscle of different groups of mice are shown in Figure 15. The Masson staining and α-SMA immunohistochemistry staining indicate increased blue collagen fibers in the D-galactose model group, darkened α-SMA staining with a larger positive area, while TBN treatment can reduce fibrosis area. Statistical analysis of the percentage of the collagen fiber area (percentage of the positive area to the total statistical area) and the percentage of α-SMA positive area (percentage of the positive area to the total statistical area) in each group of the mice is shown in Figures 16 and 17, which suggests that TBN can improve gastrocnemius muscle fibrosis in the D-galactose-induced sarcopenia model mice.

Based on the above experiments, it is found that TN-2 can also effectively improve muscle mass, muscle strength, and muscle function in sarcopenia model animals, and has the potential to be developed into drugs for the prevention or treatment of sarcopenia.

## Claims

1. A use of a ligustrazine nitrone derivative or a pharmaceutically acceptable salt thereof in the manufacture of a medicament for the prevention or treatment of sarcopenia, wherein the ligustrazine nitrone derivative has a structure of the following formula (I): 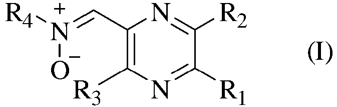 wherein, R₁ is hydrogen, methyl or 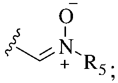 R₂ and R₃ are each independently hydrogen or a C₁-C₆ alkyl; R₄ is sec-butyl, isobutyl, tert-butyl, cyclopentyl, or cyclohexyl; R₅ is sec-butyl, isobutyl, tert-butyl, cyclopentyl, or cyclohexyl.

2. The use according to claim 1, wherein R₂ and R₃ are each independently hydrogen, methyl, ethyl, n-propyl, isopropyl, n-butyl, isobutyl, tert-butyl, or n-pentyl.

3. The use according to claim 1, wherein the ligustrazine nitrone derivative has a structure of the following formula, TBN or TN-2:

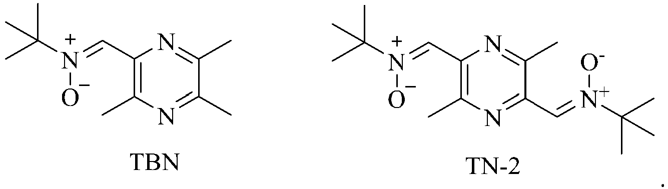

4. The use according to any one of claims 1 to 3, wherein sarcopenia is primary sarcopenia or secondary sarcopenia.

5. The use according to any one of claims 1 to 3, wherein the medicament is used for slowing down the reduction in muscle mass, maintaining or increasing muscle mass.

6. The use according to any one of claims 1 to 3, wherein the medicament is used for improving muscle strength in a patient with sarcopenia.

7. The use according to any one of claims 1 to 3, wherein the medicament is used for improving physical function in a patient with sarcopenia.

8. The use according to any one of claims 1 to 3, wherein the medicament is used for treating or preventing muscle fibrosis in a patient with sarcopenia.

9. The use according to any one of claims 1 to 3, wherein the use comprises administering 100-5000 mg of the ligustrazine nitrone derivative per day to a patient in need.

10. The use according to any one of claims 1 to 3, wherein the pharmaceutically acceptable salt is a salt formed with an inorganic acid, wherein the inorganic acid is selected from the group consisting of hydrochloric acid, hydrobromic acid, hydroiodic acid, sulfuric acid, pyrosulfuric acid, phosphoric acid, or nitric acid; or a salt formed with an organic acid, wherein the organic acid is for example methanesulfonic acid, ethanesulfonic acid, benzenesulfonic acid, p-toluenesulfonic acid, acetic acid, pyruvic acid, trifluoroacetic acid, propionic acid, hexanoic acid, benzoic acid, salicylic acid, cinnamic acid, cyclopentanone propionic acid, dodecyl sulfuric acid, 2-naphthalenesulfonic acid, citric acid, tartaric acid, stearic acid, lactic acid, oxalic acid, succinic acid, malic acid, fumaric acid, D-gluconic acid, and aspartic acid.
